# EUROPEAN PATENT APPLICATION

(11) **EP 4 625 433 A1**
(43) Date of publication of application: **01.10.2025**
(21) Application number: 25154879.8
(22) Date of filing: 30.01.2025
(51) Int. Cl.: G16H 40/20

(54) **INFORMATION OUTPUT PROGRAM, INFORMATION OUTPUT METHOD, AND INFORMATION PROCESSING DEVICE**

(30) Priority: 29.03.2024 JP 2024057870
(71) Applicant: FUJITSU LIMITED, Kawasaki-shi, Kanagawa 211-8588 (JP)
(72) Inventor: Nakao, Manabu, Kawasaki-shi, Kanagawa, 211-8588 (JP); Kuraki, Kensuke, Kawasaki-shi, Kanagawa, 211-8588 (JP); Inomata, Akihiro, Kawasaki-shi, Kanagawa, 211-8588 (JP)
(74) Representative: Hoffmann Eitle

(57) **Abstract**

An information output program for causing a computer to execute a process includes: identifying a flow of persons in a route that includes a plurality of options by using a behavior selection model that indicates which behavior a person selects for a policy; generating information that indicates a prediction result of the policy based on an identified flow of persons; and outputting generated information of a prediction result of policy to a display screen.

## Description

### FIELD

The embodiment discussed herein is related to an information output program, an information output method, and an information processing device.

### BACKGROUND

As one of workflows, a flow graph of policy is known in which a flow of allocating an object serving as the target of a policy, for example, a human or the like, to a service or the like for achieving a purpose of the policy is schematized in various fields such as medical care, nursing care, and administration.

For example, by counting a route of a flow graph of policy from the start node to a terminal node via a conditional branch including a plurality of options for each person to be the target of the policy, an effect of the policy is predicted.

In such conditional branch, as one aspect, quantitative data such as examination values of a medical checkup or categorical data such as gender is used for condition determination.

Japanese Laid-open Patent Publication No. 2024-1987, Japanese Laid-open Patent Publication No. 2019-197372, U.S. Patent Application Publication No. 2019/0180868, and U.S. Patent Application Publication No. 2018/0039949 are disclosed as related art.

### PROBLEMS

However, in a conditional branch of the above flow graph of policy, since conditional determination may be executed only with quantitative data or categorical data, it is difficult to incorporate, into an option of the conditional branch, a behavior of which selection may be changed with a policy of prompting a person to change a behavior.

In one aspect, it is an object to provide an information output program, an information output method, and an information processing device that may incorporate behavior selection of a person into an option of conditional branch.

### [Effects of Invention]

According to one embodiment, behavior selection of a person may be incorporated into an option of conditional branch.

### SUMMARY

According to an aspect of the embodiments, an information output program for causing a computer to execute a process includes: identifying a flow of persons in a route that includes a plurality of options by using a behavior selection model that indicates which behavior a person selects for a policy; generating information that indicates a prediction result of the policy based on an identified flow of persons; and outputting generated information of a prediction result of policy to a display screen.

### BRIEF DESCRIPTION OF DRAWINGS

FIG. 1 is a block diagram illustrating a functional configuration example of a server device;
FIG. 2 is a diagram exemplifying a flow graph of policy;
FIG. 3 is a diagram illustrating a specific example of a flow graph of policy;
FIG. 4 is a diagram illustrating an example of a service that prompts a behavior change;
FIG. 5 is a diagram illustrating an example of a policy flow;
FIG. 6 is a schematic diagram illustrating a setting example of a behavior selection model;
FIG. 7 is a diagram illustrating an example of model information;
FIG. 8 is a schematic diagram for describing an aspect of a graph search;
FIG. 9 is a schematic diagram for describing an example of information output;
FIG. 10 is a flowchart illustrating a procedure of information output processing;
FIG. 11 is a schematic diagram (1) for describing an application example of information output;
FIG. 12 is a schematic diagram (2) for describing an application example of information output;
FIG. 13 is a schematic diagram (3) for describing an application example of information output;
FIG. 14 is a schematic diagram for describing an application example of a behavior selection model; and
FIG. 15 is a diagram illustrating a hardware configuration example.

### DESCRIPTION OF EMBODIMENTS

Hereinafter, exemplary embodiments for implementing an information output program, an information output method, and an information processing device according to the present disclosure will be described with reference to the attached drawings. The exemplary embodiments merely indicate examples and aspects. The structures, behaviors, functions, properties, characteristics, methods, applications, and the like according to the present disclosure are not limited by such exemplification.

### <Exemplary Embodiment 1>

### <System Configuration>

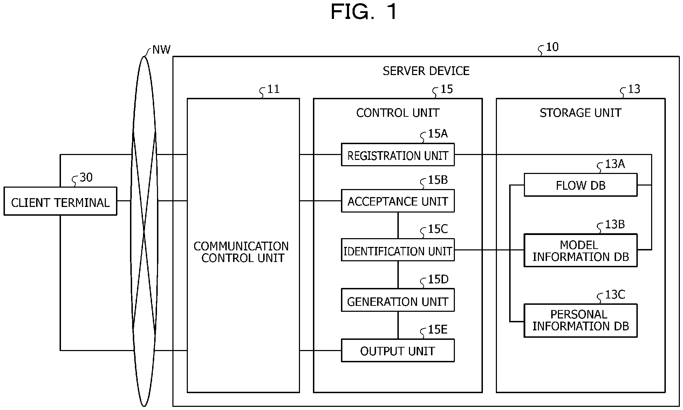

FIG. 1 is a block diagram illustrating a functional configuration example of a server device 10. FIG. 1 illustrates the server device 10 that provides an information output function in which a prediction result of an effect of a policy is output by incorporating behavior selection of a person into an option of a conditional branch in a flow graph of policy.

The server device 10 may provide the above information output function as a cloud service by executing platform as a service (PaaS) type middleware or a software as a service (SaaS) type application.

As illustrated in FIG. 1, the server device 10 may be communicably coupled to a client terminal 30 through a network NW. For example, the network NW may be arbitrary type of communication network, regardless of wired or wireless, such as the Internet or a local area network (LAN). While an example in which one client terminal 30 is coupled to one server device 10 is given in FIG. 1, arbitrary number of client terminals 30 may be coupled.

The client terminal 30 is a terminal device that receives provision of the above information output function. For example, the client terminal 30 may be used by a customer such as a policy planner as an example of a person concerned in an entity that implements a policy such as, for example, a municipality. By way of example, the client terminal 30 may be realized by arbitrary computer such as a smartphone, a tablet terminal, or a wearable terminal, in addition to a personal computer.

While an example has been given in which the above information output function is provided as a cloud service, this is not the only case. For example, the above information output function may be provided on-premise. While an example has been given in which the above information output function is provided in a client server system, this is not the only case. For example, the above information output function may be provided in a stand-alone manner by an application that runs on the client terminal 30 causing the client terminal 30 to execute processing corresponding to the above information output function.

### <Policy Model>

A "policy model" includes a diagram constituted by a plurality of components that defines the contents related to a policy in a hierarchical structure. For example, a policy model may be realized as a social concept, an organization chart, a medical guideline, or the like, in addition to a flow graph of policy.

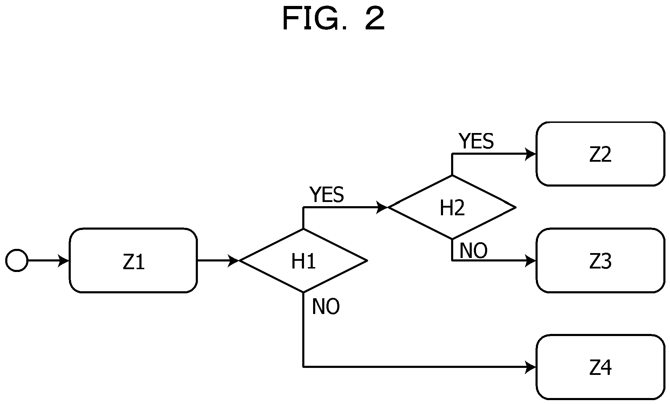

Hereinafter, as one of policy models, a flow graph of policy will be described as an example. FIG. 2 is a diagram exemplifying a flow graph of policy. For example, Z1, Z2, Z3, and Z4 illustrated in FIG. 2 indicate services implemented for a user by an administrator. These services may be referred to as "service implementation components". Specific examples of a service include, for example, in the example of the medical field, "intervention" in which an object serving as a target of a policy, for example, a resident or the like, is allocated such as reception of a medical checkup or medical examination by a specialist, and "no intervention" such as follow-up observation, but the service is not limited to policies in the medical field.

For example, H1 and H2 indicate conditional branches including a condition. These conditional branches may be referred to as "conditional branch components". Specific examples of a condition include, for example, in the example of the medical field, an estimated glomerular filtration rate (eGFR) being less than a threshold, a hemoglobin A1c value (HbA1c) being less than a threshold, and a urine protein value being a threshold or larger, but the condition is not limited to conditions in the medical field.

Z1, Z2, Z3, Z4, H1, and H2 may each be referred to as a "component". From the aspect as graph data, such "component" may correspond to an example of a "node". Coupling between nodes may correspond to an example of an "edge" including a "directed edge" and the like.

While policy planning in the medical field is described as an example in the present exemplary embodiment, use cases of the above information output function are not limited thereto. For example, the above information output function may be applied to various kinds of policy planning such as work, a test, and a questionnaire in addition to the traffic field such as road pricing and the energy field such as decarbonization and power supply. Also in such case, behavior and effect similar to those obtained in the case where the function is applied to policy planning in the medical field may be obtained.

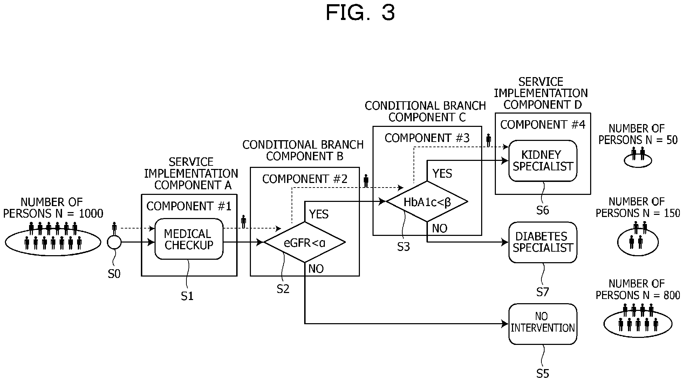

FIG. 3 is a diagram illustrating a specific example of a flow graph of policy. As illustrated in FIG. 3, a policy is modeled as a workflow constituted by a combination of components such as conditional branch and service implementation. The number of persons who receive each service is output from a model learned by accumulating the information on the flow of persons and the parameters from the actual result values obtained when the service is used for each conditional branch component.

In the example illustrated in FIG. 3, in reference sign S0, the number of persons N = 1000 is input. In reference sign S1, "medical checkup" is set for component #1 as service implementation component A. In reference sign S2, "eGFR < α" is set for component #2 as conditional branch component B. When "eGFR < α" is not satisfied (see NO route of reference sign S2), as indicated by reference sign S5, it is determined that there is "no intervention" of a specialist in the citizen.

On the other hand, when "eGFR < α" is satisfied (see YES route of reference sign S2), as indicated by reference sign S3, "HbA1c < β" is set for component #3 as conditional branch component C. When "HbA1c < β" is satisfied (see YES route of reference sign S3), as indicated by reference sign S6, "kidney specialist" is set for component #4 as conditional branch component D, and it is determined that intervention of a "kidney specialist" has to be made in the citizen. On the other hand, when "HbA1c < β" is not satisfied (see NO route of reference sign S3), as indicated by reference sign S7, it is determined that intervention of a "diabetes specialist" has to be made in the citizen.

In the example illustrated in FIG. 3, as indicated by arrows, the number of persons flowing in the order of component #1, component #2, component #3, and component #4 is predicted. For example, a result of allocating the number of persons N = 1000 to Z2 to Z4 as interventions in the flow graph of policy illustrated in FIG. 3 is as follows. 50 persons are allocated to intervention Z2. 150 persons are allocated to intervention Z3. 800 persons are allocated to intervention Z4.

Hereinafter, there are cases in which a flow graph of policy is simply referred to as a "policy flow". A policy flow may be shared in arbitrary framework. By way of example only, a policy flow may be shared among organizations around the world, for example, public organizations such as municipalities, through a platform of data infrastructure capable of sharing, cross-referencing, and updating policy flows.

Such platform of data infrastructure may be provided by a business operator that provides the above information output function, or may be provided by another business operator. For example, a policy planner may refer to the policy flows around the world collected in the above data infrastructure through the client terminal 30. At this time, by incorporating all or part of the policy flows collected in the data infrastructure, an existing policy flow may be updated and planning of an original policy flow may be supported.

In addition, the above data infrastructure may provide not only sharing of policy flows but also the following backend functions. For example, a simulation may be executed in which a flow of an object that is a target of a service is simulated in existing and original policy flows. In addition, each indicator in existing and original policy flows, for example, effect, cost, or the like may be evaluated, and comparison between existing and original policy flows or comparison between a plurality of original policy flows may be executed.

### <One Aspect of Problem>

As has been described in the above background section, in a conditional branch of the policy flow according to the above related art, since conditional determination may be executed only with quantitative data or categorical data, it is difficult to incorporate, into an option of the conditional branch, a behavior of which selection may be changed with a policy of prompting a person to change a behavior.

As one of cases where incorporating behavior selection into an option of conditional branch of a policy flow as described above has a technical value, planning of a health policy is given as an example.

With a view to extending the healthy life expectancy of people, countries are requesting local governments and companies to formulate data health plans and implement efficient health businesses.

In response to such request, insurers such as companies implement health businesses such as medical checkup reception recommendation and health coaching, and plan and execute various policies every year in order to achieve the target values of the formulated data health plans.

For example, in order to improve a medical checkup reception rate, various services such as assistance of medical checkup expenses, support of medical checkup reservation by a call center, and recommendation using various channels such as postcard, email, and social networking service (SNS) are provided.

These services are reviewed for improvement, and there is high demand for estimating in advance how much the reception rate will be improved when a service is changed and determining how a service is changed based on cost-effectiveness or the like.

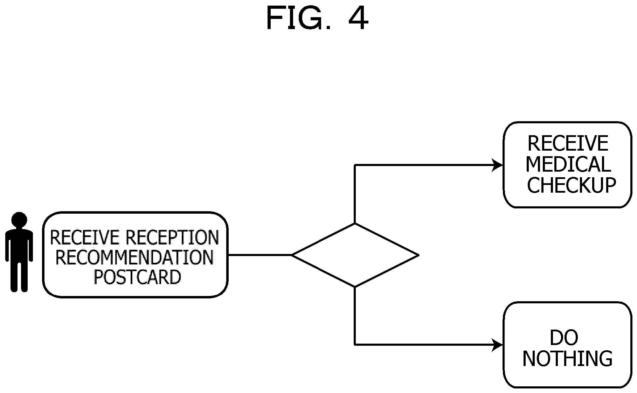

FIG. 4 is a diagram illustrating an example of a service that prompts a behavior change. As illustrated in FIG. 4, reception recommendation of a medical checkup prompts a recipient to take the behavior of receiving a medical checkup. Whether a person who has received a postcard of this reception recommendation selects a behavior of actually receiving a medical checkup or selects a behavior of doing nothing is left to individual determination of the person who has received the postcard.

There is a high technical value in the prediction of the effect of an increase in the number of medical checkup receptions in a policy flow in which a conditional branch in which the behavior of a person changes by changing the message in such postcard of reception recommendation is incorporated.

### <One Aspect of Problem Solving Approach>

The information output function according to the present exemplary embodiment achieves incorporation of behavior selection of a person into an option of conditional branch by predicting a route to be allocated by an option of a conditional branch of a policy flow using a behavior selection model associated with the conditional branch and outputting the flow of persons in the policy flow.

### <Configuration of Server Device 10>

Next, a functional configuration of the server device 10 that provides the above information output function will be described. FIG. 1 schematically illustrates blocks related to the information output function of the server device 10. As illustrated in FIG. 1, the server device 10 includes a communication control unit 11, a storage unit 13, and a control unit 15. Functional units related to the above information output function are merely extracted and illustrated in FIG. 1, and functional units other than those illustrated may be included in the server device 10.

The communication control unit 11 is a functional unit that controls communication with another device such as the client terminal 30. As one aspect, the communication control unit 11 may be realized by a network interface card such as a LAN card. As one aspect, the communication control unit 11 accepts a request for requesting information output related to the effect of a policy from the client terminal 30, or outputs a prediction result of the effect of a policy to the client terminal 30.

The storage unit 13 is a functional unit that stores various types of data. As one aspect, the storage unit 13 may be realized by an internal, external, or auxiliary storage of the server device 10. For example, the storage unit 13 stores a flow database (DB) 13A, a model information DB 13B, and a personal information DB 13C. Description of the flow DB 13A, the model information DB 13B, and the personal information DB 13C will be made later together with the description of reference or registration.

The control unit 15 is a functional unit that performs overall control of the server device 10. For example, the control unit 15 may be realized by a hardware processor. As illustrated in FIG. 1, the control unit 15 includes a registration unit 15A, an acceptance unit 15B, an identification unit 15C, a generation unit 15D, and an output unit 15E. The control unit 15 may be realized by a hard wired logic or the like.

The registration unit 15A is a processing unit that registers a policy flow. As one aspect, the registration unit 15A newly registers a policy flow created by the client terminal 30 in the flow DB 13A stored in the storage unit 13, or saves a policy flow in which the policy flow registered in the flow DB 13A is edited by overwriting.

A set of such policy flows managed by the flow DB 13A may include templates of existing policies around the world collected in the above data infrastructure. For example, when a policy is planned, whether a similar policy has been implemented in the past is emphasized from the aspect of administrative (political) ease of execution. From such aspect, an original plan may be updated by incorporating all or part of the existing policies similar to the original plan of a policy planner among the templates collected in the data infrastructure.

In a case where a policy flow is created in which behavior selection of a person is incorporated in an option of a conditional branch, setting of a behavior selection model is performed in the policy flow in which a service that prompts a person to change a behavior is associated with an option of behavior in a conditional branch.

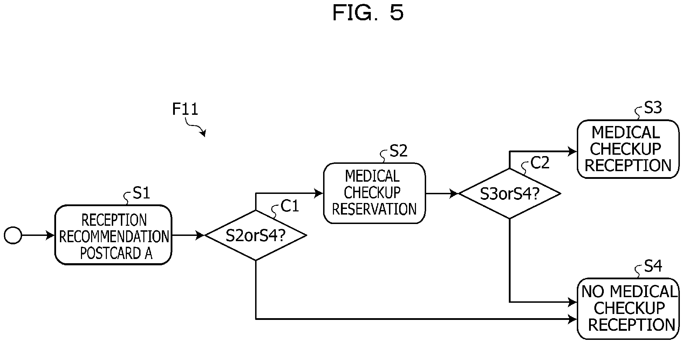

FIG. 5 is a diagram illustrating an example of a policy flow. FIG. 5 illustrates a policy flow F11 created by changing a part of an existing policy flow f1 registered in the flow DB 13A. As illustrated in FIG. 5, the policy flow F11 includes, as examples of a service that prompts a person to change a behavior, service S1 of sending a reception recommendation postcard A and service S2 of accepting a medical checkup reservation.

Of these services in the policy flow F11, a change is added to the message of a reception recommendation postcard O sent in service S1 in the existing policy flow f1. For example, a change is made in service S1 of the policy flow F11 such that the reception recommendation postcard A is sent in which "you may receive a medical checkup for free" is added to the default text of the message of the reception recommendation postcard O sent in service S1 of the existing policy flow f1.

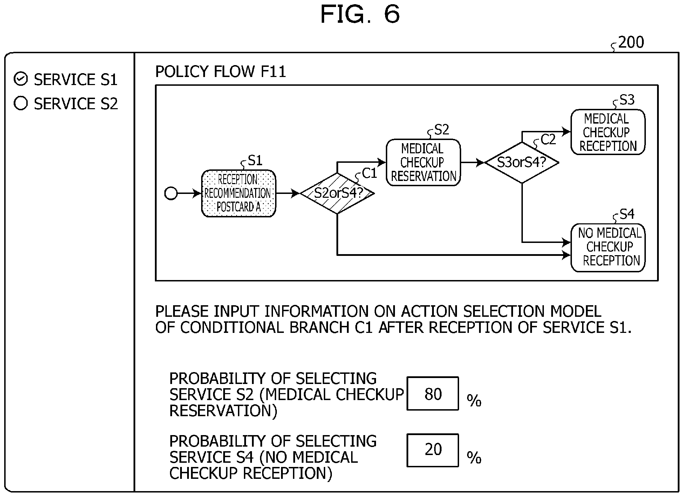

FIG. 6 is a schematic diagram illustrating a setting example of a behavior selection model. FIG. 6 exemplifies a setting screen 200 in which service S1 (reception recommendation postcard A) of the policy flow F11 illustrated in FIG. 5 is designated as the service that affects behavior selection, and conditional branch C1 of the policy flow F11 illustrated in FIG. 5 is designated as the conditional branch related to behavior selection.

In the setting screen 200, as an example of an algorithm of behavior selection model, an example is illustrated in which a simple behavior model is selected that generates a random number corresponding to an index of each option in accordance with a probability that the option is selected for each option of a conditional branch.

In the case where such simple behavior model is used, in the setting screen 200, a probability of selecting service S2 (medical checkup reservation) and a probability of selecting service S4 (no medical checkup reception) may be accepted. For example, in the example of the setting screen 200 illustrated in FIG. 6, a behavior selection model that realizes an estimation of the expected effect that 80% of the persons who have received service S1 select service S2, while 20% of the persons who have received service S1 select service S4, is associated with service S1.

In this case, the registration unit 15A additionally registers model information 13B1, the input of which has been accepted through the setting screen 200, in the model information DB 13B stored in the storage unit 13.

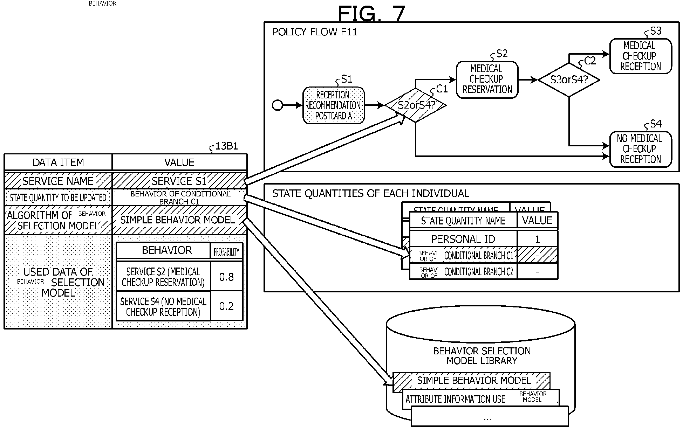

FIG. 7 is a diagram illustrating an example of the model information 13B1. As illustrated in FIG. 7, the model information 13B1 may be data in which a service name, a state quantity to be updated, an algorithm of behavior selection model, and used data of behavior selection model are associated with each other. According to this model information 13B1, a behavior selection model is set which is associated with service S1 of the policy flow F11 and which selects the behavior of conditional branch C1 from among the state quantities of a person to whom the policy flow F11 is applied. The simple behavior model is set as the algorithm of behavior selection model. In the probability distribution used by the simple behavior model for generation of a random number, the probability of selecting service S2 (medical checkup reservation) of "80%" is set, and the probability of selecting service S4 (no medical checkup reception) of "20%" is set.

While FIG. 6 and FIG. 7 illustrate an example in which the simple behavior model is used as an example of the algorithm of behavior selection model, other algorithms may be used such as an attribute information use behavior model in which attribute information is used for behavior selection and a discrete choice model.

Returning to the description of FIG. 1, the acceptance unit 15B is a processing unit that accepts various types of information from the client terminals 30. As one aspect, the acceptance unit 15B may accept, from the client terminal 30, a request that requests information output related to an effect of a policy. When such information output request is accepted, the acceptance unit 15B may accept designation of one or a plurality of policy flows. For example, from an aspect of achieving comparison of effects between a plurality of policies, an existing policy flow and an original policy flow to be planned may be designated, or a plurality of original policy flows may be designated.

The identification unit 15C is a processing unit that identifies a flow of persons in a route including a plurality of options by using a behavior selection model indicating which behavior a person selects for a policy. As one aspect, the identification unit 15C executes the following processing for each of the M policy flows designated by the information output request accepted by the acceptance unit 15B. For example, the identification unit 15C inputs, to the m-th policy flow, personal information, for example, personal attribute information such as age, gender, and medical checkup results, of a group as the target of the policy, for example, insured persons covered by health insurance, in the personal information DB 13C stored in the storage unit 13. With this, the identification unit 15C identifies, for each individual included in the group as the target of the policy, a route to which the individual is allocated from the start node of the policy flow to a terminal node via conditional branch nodes.

For example, the identification unit 15C executes, for each of N individuals included in the group as the target of the policy, a graph search that searches for a node by following edges while prioritizing the depth direction of the policy flow until a terminal node is reached from the start node. For example, the identification unit 15C causes the n-th person to transition to the next node. At this time, in a case where model information including the service name corresponding to the node after transition in the data entry exists in the model information DB 13B, the identification unit 15C generates a behavior selection model in accordance with the settings defined in the model information. The identification unit 15C selects one option of behavior from the options of behavior in the next conditional branch using the behavior selection model. After that, the identification unit 15C executes update of registering the option of behavior selected by the behavior selection model in the state quantities of the n-th person as the behavior in the next conditional branch. In a case where the node after transition is a conditional branch, the identification unit 15C sets, as a course, an edge corresponding to the option of behavior held as a behavior of conditional branch in the state quantities of the n-th person. Such graph search is repeated until a terminal node is reached.

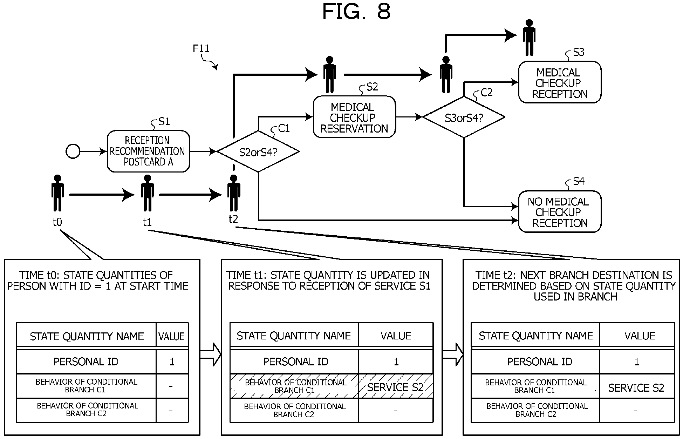

FIG. 8 is a schematic diagram for describing an aspect of a graph search. FIG. 8 exemplifies a graph search related to a person identified by personal ID "1", and extracts and illustrates the state quantities of personal ID "1" at times t0, t1, and t2.

As illustrated in FIG. 8, time t0 corresponds to the start time. At time t0, the person with personal ID "1" is located at the start node, which is the initial position.

Next, at time t1, the person with personal ID "1" transitions to service S1 (reception recommendation postcard A). At time t1, an option of behavior in the next conditional branch C1 is selected using the behavior selection model generated in accordance with the settings defined in the model information 13B1 illustrated in FIG. 7 For example, in the example of the model information 13B1 illustrated in FIG. 7, in the behavior selection model, 80% of the persons who have received service S1 select service S2, while 20% of the persons who have received service S1 select service S4. FIG. 8 illustrates an example in which the behavior selection model selects service S2 (medical checkup reservation) as the option of behavior in the next conditional branch C1. In this case, as illustrated by hatching in FIG. 8, the identification unit 15C executes update of registering service S2 (medical checkup reservation) selected by the behavior selection model in the state quantities of the person with personal ID "1" as the behavior in the next conditional branch C1.

At time t2, the person with personal ID "1" transitions to conditional branch C1. At this time t2, an edge corresponding to service S2 (medical checkup reservation) held as a behavior of conditional branch C1 in the state quantities of the person with personal ID "1" is set as a course. With this, at the next time t3, the person with personal ID "1" may make a transition to service S2 (medical checkup reservation).

After that, the graph search is executed after time t3 in a similar manner until a terminal node is reached. As a result of such graph search, the route that passes through each node of service S1, conditional branch C1, service S2, conditional branch C2, and service S3 is obtained as a route to which the person with personal ID "1" is allocated by the policy flow F11.

The generation unit 15D is a processing unit that generates information indicating a prediction result of an effect of a policy based on the flow of persons identified by the identification unit 15C. As one aspect, the generation unit 15D executes the following processing for each of the M policy flows designated by the information output request accepted by the acceptance unit 15B. For example, the generation unit 15D adds up, for each edge included in the m-th policy flow, the number of persons who pass through the edge based on the route in the m-th policy flow allocated to each individual by the identification unit 15C. After that, the generation unit 15D sets the display form of the edge, for example, thickness, according to the number of persons who have passed through the edge included in the m-th policy flow. For example, while a thick edge may be set as the number of passing persons increases, a thin edge may be set as the number of passing persons decreases. In this way, the generation unit 15D generates, for each of the M policy flows, display data in which the thickness of each edge is set according to the number of persons who have passed through each edge.

The output unit 15E executes output control for the client terminal 30. As one aspect, the output unit 15E causes the client terminal 30 to display the display data of M policy flows generated by the generation unit 15D as a response to the information output request accepted by the acceptance unit 15B.

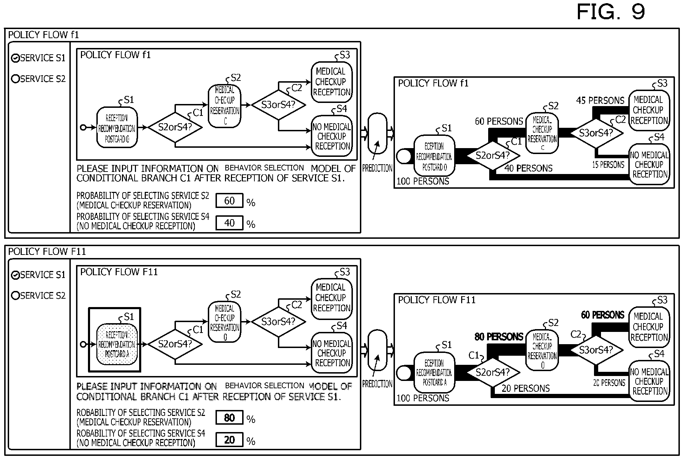

FIG. 9 is a schematic diagram for describing an example of information output. FIG. 9 illustrates a display example of the policy flow f1 including service S1 of sending the reception recommendation postcard O in a default text and the policy flow F11 including service S1 of sending the reception recommendation postcard A in which a message change of adding "you may receive a medical checkup for free" to the default text is performed.

As illustrated in FIG. 9, in the policy flow f1, 60% of the persons who have received service S1 select service S2, while 40% of the persons who have received service S1 select service S4. On the other hand, in the policy flow F11, 80% of the persons who have received service S1 select service S2, while 20% of the persons who have received service S1 select service S4.

It is possible to estimate how much effect may be expected from such change in behavior selection for the improvement of the reception rate of medical checkup, which is the target of a policy. For example, in the example illustrated in FIG. 9, the number of persons "45" is displayed in association with the edge connecting conditional branch C2 and service S3 in the policy flow f1, while the number of persons "60" is displayed in association with the edge connecting conditional branch C2 and service S3 in the policy flow F11. This allows estimation of the effect that the number of persons receiving a medical checkup is increased by 15, for example, the effect that the reception rate of medical checkup is increased by 1.3 times, by changing the reception recommendation postcard O to the reception recommendation postcard A.

### <Flow of Processing>

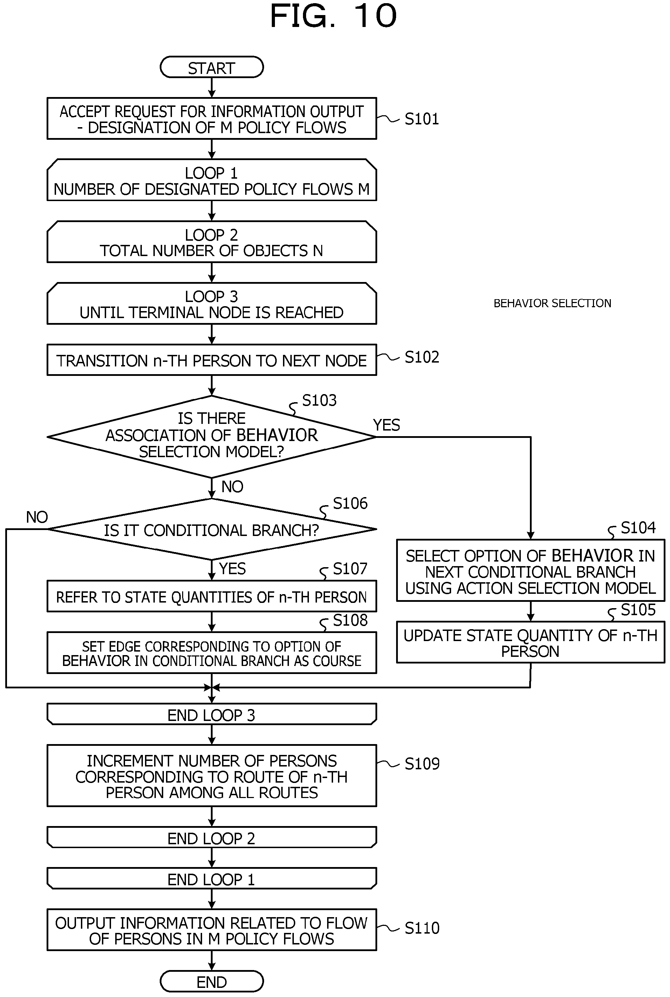

Next, a flow of processing of the server device 10 according to the present exemplary embodiment will be described. FIG. 10 is a flowchart illustrating a procedure of information output processing. By way of example only, this processing may be started in a case where an information output request is accepted by the acceptance unit 15B. The condition for starting this processing is not limited to the time when the information output request is accepted, and may be a condition that a new policy flow is registered or an existing policy flow is saved by overwriting.

As illustrated in FIG. 10, when an information output request is accepted (step S101), the identification unit 15C executes loop processing 1 of repeating the processing of the following step S102 to the following step S109 by the number of times corresponding to the number M of policy flows designated by the information output request accepted in step S101. The processing of the following step S102 to the following step S109 may be executed in parallel for each of the M policy flows.

The identification unit 15C executes loop processing 2 of repeating the processing of the following step S102 to the following step S109 by the number of times corresponding to the total number of persons N applied to the graph search of the m-th policy flow. The processing of the following step S102 to the following step S109 may be executed in parallel for each of the M policy flows.

The identification unit 15C executes loop processing 3 of repeating the processing of the following step S102 to the following step S108 until the graph search of the n-th person reaches a terminal node of the m-th policy flow. The processing of the following step S102 to the following step S108 may be executed in parallel for each of the M policy flows.

For example, the identification unit 15C causes the n-th person to transition to the next node (step S102). At this time, in the case where model information including the service name corresponding to the node after transition in the data entry exists in the model information DB 13B (Yes in step S103), the identification unit 15C executes the following processing.

For example, the identification unit 15C selects one option of behavior from the options of behavior in the next conditional branch using the behavior selection model generated in accordance with the settings defined in the model information (step S104). After that, the identification unit 15C executes update of registering the option of behavior selected in step S104 in the state quantities of the n-th person as the behavior in the next conditional branch (step S105).

When the node after transition is a conditional branch (No in step S103 and Yes in step S106), the identification unit 15C refers to the state quantities of the n-th person (step S107). The identification unit 15C sets, as a course, an edge corresponding to the option of behavior held as a behavior of conditional branch in the state quantities of the n-th person (step S108).

By repeating such loop processing 3, a route to which the n-th person is allocated by the m-th policy flow may be searched for.

After that, the generation unit 15D increments the number of persons passing through the edge overlapping the route to which the n-th person is allocated by the m-th policy flow among the edges included in the m-th policy flow (step S109).

By repeating such loop processing 2, the routes of N persons and the number of persons passing through each edge in the m-th policy flow are obtained. Consequently, display data of the m-th policy flow is generated.

By repeating the loop processing 1, display data is generated for each of the M policy flows.

After that, the output unit 15E causes the client terminal 30 to display the display data of M policy flows generated as a result of the loop processing 1 to the loop processing 3 (step S110), and ends the processing.

### <One Aspect of Effects>

As has been described above, the server device 10 according to the present exemplary embodiment predicts a route to be allocated by an option of a conditional branch of a policy flow using a behavior selection model associated with the conditional branch, and outputs the flow of persons in the policy flow. Therefore, according to the server device 10 according to the present exemplary embodiment, behavior selection of a person may be incorporated into an option of conditional branch.

### <Exemplary Embodiment 2>

While an exemplary embodiment of the present disclosure has been described, various applications are possible, and the present disclosure may be implemented in various different forms other than the above-described exemplary embodiment.

### <Exhibition of Creativity>

Matters described in the above exemplary embodiment 1, for example, specific examples of a policy flow as the target of information output, the type of algorithm of behavior selection model, and the like, are merely examples and may be changed. The order of processing in the flowchart described in the exemplary embodiment may also be changed within a range without contradiction.

### <Application Example 1 of Information Output>

While the policy flow f1 of sending the reception recommendation postcard O including the message of a default text and the policy flow F11 of sending the reception recommendation postcard A in which a message change of adding "you may receive a medical checkup for free" to the default text is performed are exemplified as a change example of service S1 in the above exemplary embodiment 1, a policy flow of sending a reception recommendation postcard in which a different message change is performed may be further displayed.

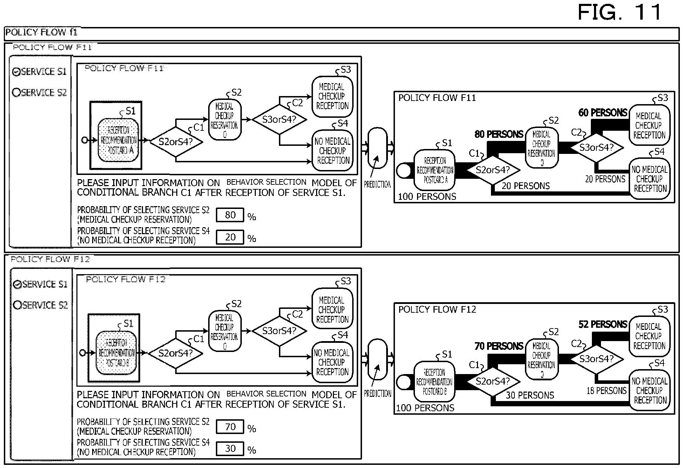

FIG. 11 is a schematic diagram (1) for describing an application example of information output. For convenience of description, display of the policy flow f1, which has already been described with reference to FIG. 9, is compressed in FIG. 11. As illustrated in FIG. 11, in addition to the policy flow f1 and the policy flow F11 illustrated in FIG. 9, a policy flow F12 is displayed that includes service S1 of sending a reception recommendation postcard B in which a message change of adding "anyone has a possibility of developing cancer, so receive a medical checkup" to the default text is performed.

In this policy flow F12, 70% of the persons who have received service S1 select service S2, while 30% of the persons who have received service S1 select service S4.

By displaying such policy flow F12, the policy flow f1, the policy flow F11, and the policy flow F12 may be compared as follows. For example, the number of persons "45" is displayed in association with the edge connecting conditional branch C2 and service S3 in the policy flow f1. On the other hand, the number of persons "60" is displayed in association with the edge connecting conditional branch C2 and service S3 in the policy flow F11, and the number of persons "52" is displayed in association with the edge connecting conditional branch C2 and service S3 in the policy flow F12. Consequently, such evaluation is possible that both of the proposed message changes of the reception recommendation postcard A and the reception recommendation postcard B are more effective in the improvement of the reception rate of medical checkup than the default text. It may be also determined that the reception recommendation postcard A is more effective in the reception rate of medical checkup compared to the reception recommendation postcard B.

### <Application Example 2 of Information Output>

While an example in which service S1 is changed has been given in the above exemplary embodiment 1, changing of service S1 is not the only case, and similar information output may be achieved in a case where a different service is changed.

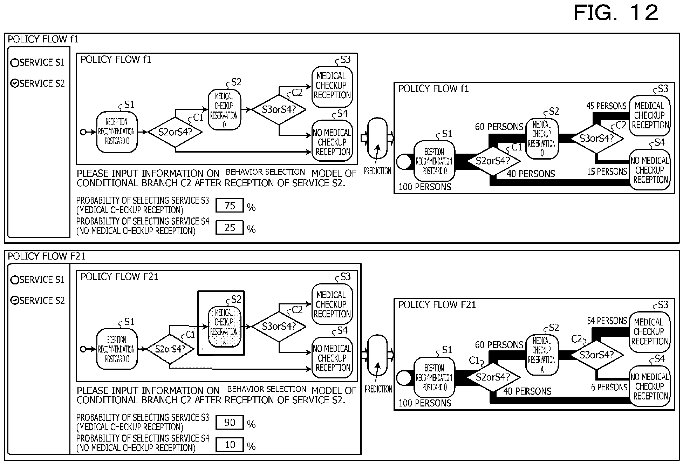

FIG. 12 is a schematic diagram (2) for describing an application example of information output. FIG. 12 illustrates a display example of the policy flow f1 including service S2 in which the current reservation system O is used as the reservation system of medical checkup and a policy flow F21 including service S2 in which the reservation system of medical checkup is changed to reservation system A.

As illustrated in FIG. 12, in the policy flow f1, 75% of the persons who have received service S2 select service S3, while 25% of the persons who have received service S2 select service S4. On the other hand, in the policy flow F21, 90% of the persons who have received service S2 select service S3, while 10% of the persons who have received service S2 select service S4.

It is possible to estimate how much effect may be expected from such change in behavior selection for the improvement of the reception rate of medical checkup, which is the target of a policy. For example, in the example illustrated in FIG. 12, the number of persons "45" is displayed in association with the edge connecting conditional branch C2 and service S3 in the policy flow f1, while the number of persons "54" is displayed in association with the edge connecting conditional branch C2 and service S3 in the policy flow F21. This allows estimation of the effect that the number of persons receiving a medical checkup is increased by nine, for example, the effect that the reception rate of medical checkup is increased by 1.2 times, by changing the reservation system for medical checkup from reservation system O to reservation system A.

### <Application Example 3 of Information Output>

While examples in which the policy flow F12 and the policy flow F21 are displayed as two proposals of change related to the same type of service S1 have been given in the above application examples, proposals of change for different services may be displayed.

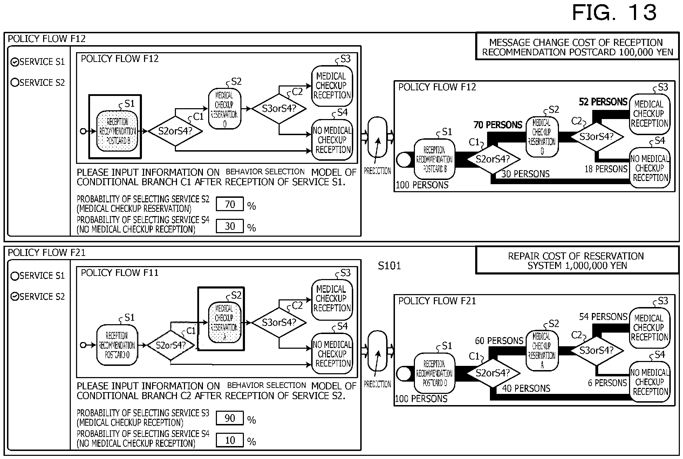

FIG. 13 is a schematic diagram (3) for describing an application example of information output. FIG. 13 illustrates a display example of the policy flow F12 including service S1 of sending the reception recommendation postcard B in which a message change of adding "anyone has a possibility of developing cancer, so receive a medical checkup" to the default text is performed and the policy flow F21 including service S2 in which the reservation system of medical checkup is changed to reservation system A. In FIG. 13, the cost of changing service S1 "100,000 yen" is displayed in association with the policy flow F12, and the cost of changing service S2 "1,000,000 yen" is displayed in association with the policy flow F21.

For example, in the example illustrated in FIG. 13, the number of persons "52" is displayed in association with the edge connecting conditional branch C2 and service S3 in the policy flow F11, and the number of persons "54" is displayed in association with the edge connecting conditional branch C2 and service S3 in the policy flow F21. Consequently, it is visualized that the number of persons receiving a medical checkup (reception rate) is higher in the policy flow F21, which is a proposal of changing service S2, than in the policy flow F12, which is a proposal of changing service S1. By visualization of the cost of the proposal of changing service S1 "100,000 yen" and the cost of the proposal of changing service S2 "1,000,000 yen", cost-effectiveness, so-called cost performance, may be presented. For example, in a case where a policy is selected with cost emphasized, the policy flow F12, which is a proposal of changing service S1, may be adopted.

### <Application Example of Behavior Selection Model>

While an example in which the simple behavior model is used has been given as an example of the algorithm of behavior selection model in the above exemplary embodiment 1, an attribute information use behavior model in which attribute information is used for behavior selection may be used.

For example, while an example in which each individual selects a behavior with the same probability has been given in the simple behavior model, the probability of selecting a behavior of conditional branch may be changed according to personal attribute information, for example, the age or the like in the attribute information use behavior model.

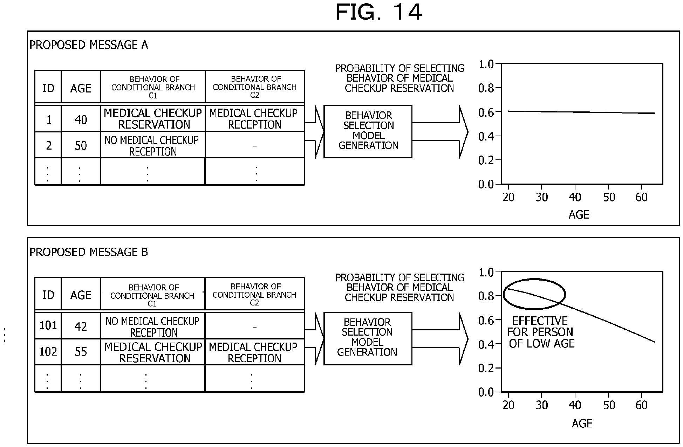

Such personal attribute information may be obtained as statistical data through an experiment, for example, a sampling survey or the like. FIG. 14 is a schematic diagram for describing an application example of a behavior selection model. FIG. 14 illustrates an example in which a behavior selection model is constructed by using, as explanatory variables, data obtained from an experiment or the like on whether a behavior of reserving a medical checkup is changed for the two proposed message changes of the reception recommendation postcard A and the reception recommendation postcard B, for example, personal attributes such as age.

FIG. 14 illustrates experimental data 20A in a case where reception recommendation is made with a proposed message change A in which "you may receive a medical checkup for free" is added to the default text, and experimental data 20B in a case where reception recommendation is made with a proposed message change B in which "anyone has a possibility of developing cancer, so receive a medical checkup" is added to the default text. By using these experimental data 20A and experimental data 20B, logistic regression analysis is executed with age as an explanatory variable and whether a reservation of medical checkup is made as an objective variable.

Consequently, a behavior selection model 21A may be generated from the experimental data 20A, and a behavior selection model 21B may be generated from the experimental data 20B. For example, in the case of the experimental data 20A, the behavior selection model 21A is generated in which a behavior of making a reservation of medical checkup is selected at a uniform probability across all ages. On the other hand, in the case of the experimental data 20B, the behavior selection model 21B is generated in which the probability of selecting the behavior of making a reservation of medical checkup increases as the age decreases.

While an example in which logistic regression is used has been given as an example of the attribute information use behavior model, in the case of the discrete choice model, the attribute information use behavior model may be realized by formulating a definite term corresponding to a personal attribute.

### <Digital Twin>

The server device 10 may execute a simulation that simulates a flow of objects that are targets of a service in a digital twin that virtually reproduces a real space. First, the server device 10 generates a digital twin in which a real space is reproduced in a virtual space. Next, in the generated digital twin, the server device 10 executes a simulation of a flow of persons in a route including a plurality of options using a behavior selection model. The server device 10 generates information indicating a prediction result of policy based on a result of the executed simulation.

For example, the server device 10 generates, in a virtual space, a digital twin that is time-synchronized with a real space. For example, the server device 10 collects data related to personal attribute information of a person from a terminal, and updates the personal attribute information of the person in real time in the digital twin. For example, in the digital twin, the server device 10 identifies a group of agents corresponding to persons who are present at the current time point. For example, in the digital twin, the server device 10 inputs the identified group of agents to a policy flow. The server device 10 generates information indicating a prediction result of policy based on the input result of the policy flow.

For example, the server device 10 identifies, for each individual included in the group of agents, a route to which the individual is allocated from the start node of the policy flow to a terminal node via conditional branch nodes. For example, in the digital twin, the server device 10 executes a simulation of whether each of a plurality of persons takes a behavior for a policy, using the attribute information of each of the plurality of agents corresponding to each of the plurality of persons and the behavior selection model associated with a conditional branch of the policy model. The server device 10 identifies a route to which each of the plurality of persons is allocated in the options of a conditional branch of the policy model based on the result of simulation. The server device 10 generates information indicating a prediction result of policy including the route to which each of the plurality of identified persons is allocated. Consequently, the server device 10 may execute a simulation with high reproducibility. The server device 10 may improve the accuracy of a prediction result of policy.

### <System>

The processing procedure, control procedure, specific names, and information including various types of data and parameters indicated in the above document and drawings may be changed arbitrarily unless otherwise identified. For example, any one or more functional units of the registration unit 15A, the acceptance unit 15B, the identification unit 15C, the generation unit 15D, and the output unit 15E in the server device 10 may be configured by different devices.

Each illustrated constituent element of each device is a functional concept, and does not have to be physically configured as illustrated in the drawings. For example, the specific form of distribution or integration of each device is not limited to that illustrated in the drawings. For example, all or some of the constituent elements may be configured by being functionally or physically distributed or integrated in arbitrary units according to various types of loads, usage states, and the like. Each configuration may be a physical configuration.

All or arbitrary part of each processing function performed by each device may be realized by a central processing unit (CPU) and a program analyzed and executed by the CPU, or may be realized as hardware by wired logic.

### <Hardware>

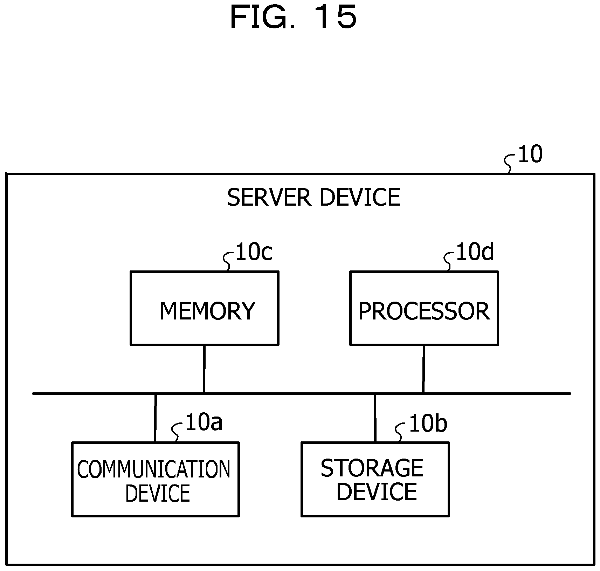

Next, a hardware configuration example of a computer described in the above exemplary embodiment will be described. FIG. 15 is a diagram illustrating a hardware configuration example. As illustrated in FIG. 15, the server device 10 includes a communication device 10a, a storage device 10b, a memory 10c, and a processor 10d. The constituent elements illustrated in FIG. 15 may be coupled to each other by a bus or the like.

The communication device 10a is a network interface card or the like. The storage device 10b is a storage device such as a hard disk drive (HDD) or a solid-state drive (SSD). For example, the storage device 10b stores a program that causes the functions illustrated in FIG. 1 to operate, a DB, and the like.

The processor 10d causes a process that executes the functions described in FIG. 1 to operate by reading, from the storage device 10b or the like, a program that executes processing similar to that of the processing units illustrated in FIG. 1 and loading the program into the memory 10c.

Such process realizes a function similar to that of a processing unit of an information processing device 10. For example, the processor 10d reads, from the storage device 10b or the like, a program having functions similar to those of the registration unit 15A, the acceptance unit 15B, the identification unit 15C, the generation unit 15D, the output unit 15E, and the like. The processor 10d executes a process that executes processing similar to that of the registration unit 15A, the acceptance unit 15B, the identification unit 15C, the generation unit 15D, the output unit 15E, and the like.

In this way, the information processing device 10 operates as an information processing device that executes an information output method by reading and executing a program. The information processing device 10 may also realize functions similar to those of the above-described exemplary embodiment by reading the above program from a recording medium with a medium reading device and executing the above read program. The program referred to in this another exemplary embodiment is not limited to that executed by the information processing device 10. For example, the present disclosure may be similarly applied to a case where another computer or a server executes a program or a case where the other computer and the server execute a program in cooperation with each other.

The above program may be distributed through a network such as the Internet. The above program may be recorded in arbitrary recording medium and executed by being read from the recording medium by a computer. For example, the recording medium may be realized by a hard disk, a flexible disk (FD), a CD-ROM, a magneto-optical (MO) disk, a Digital Versatile Disc (DVD), or the like.

The following appendices are further disclosed in relation to the embodiment including the above exemplary embodiments.

## Claims

1. An information output program for causing a computer to execute a process comprising:
identifying a flow of persons in a route that includes a plurality of options by using a behavior selection model that indicates which behavior a person selects for a policy;
generating information that indicates a prediction result of the policy based on an identified flow of persons; and
outputting generated information of a prediction result of policy to a display screen.

2. The information output program according to claim 1, wherein
the computer is caused to further execute a process of acquiring data related to the person,
the identifying includes a process of, when acquired data related to a person is input to a policy model, identifying a route to a terminal node through nodes and edges by using a behavior selection model that indicates which behavior the person selects for the policy, and
the generating includes a process of generating information that indicates a prediction result of policy related to a service to be provided to the person based on an identified route.

3. The information output program according to claim 1, wherein
the identifying includes a process of predicting a number of persons who use a service in each of a plurality of routes to terminal nodes branched with an intermediate node as a starting point, based on a behavior selection model that indicates which behavior a person selects for the policy, and
the generating includes a process of generating information that indicates a prediction result of policy in which a predicted number of persons and a service to be provided to the person are associated with each other.

4. The information output program according to claim 1, wherein
the computer is caused to further execute a process of acquiring a policy model which has a flow structure constituted by nodes and edges, and in which a behavior selection model that indicates which behavior a person selects for the policy is associated with an intermediate node of the flow structure, and a service to be provided to the person is associated with an intermediate node or a terminal node of the flow structure,
the identifying includes a process of identifying a route to a terminal node by tracing nodes and edges of the flow structure based on the behavior selection model when data related to a person to be analyzed is input to an acquired policy model,
the generating includes a process of generating information that indicates a prediction result of policy in which a number of persons flowing in an identified route and a service to be provided to the person are associated with each other, and
the outputting includes a process of outputting generated information that indicates a prediction result of policy to the display screen.

5. The information output program according to claim 1, wherein
the identifying identifies a number of persons flowing through a node in a flow graph based on the behavior selection model when acquired data related to a person is input to a policy model,
the generating generates an image in which edges are made thick based on an identified number of persons, and
the outputting displays a generated image on the display screen.

6. The information output program according to claim 1, wherein
the generating includes
a process of generating an image that indicates a first prediction result in which a number of persons flowing through a node in a flow graph is predicted, by inputting data related to a person to a first policy model before the policy is executed, and
a process of generating an image that indicates a second prediction result in which a number of persons flowing through a node in a flow graph after the policy is executed is predicted, by inputting to a second policy model after the policy is executed, and
the outputting includes a process of displaying, on the display screen, the image that indicates a first prediction result and the image that indicates a second prediction result.

7. The information output program according to claim 6, wherein
the computer is caused to further execute a process of
identifying the behavior selection model that corresponds to a policy under consideration, and
storing an identified behavior selection model in association with an intermediate node of a second policy model after the policy is executed.

8. The information output program according to claim 1, wherein
the computer is caused to further execute a process of
generating a digital twin in which a real space is reproduced in a virtual space,
executing, in the digital twin that has been generated, a simulation of a flow of persons in a route that includes the plurality of options by using the behavior selection model, and
generating information that indicates a prediction result of the policy based on a result of an executed simulation.

9. The information output program according to claim 8, wherein
the generating of a digital twin generates, in the virtual space, a digital twin that is time-synchronized with the real space,
the executing of a simulation executes, by using attribute information of each of a plurality of agents that corresponds to each of a plurality of persons and the behavior selection model associated with a conditional branch of a policy model, in the digital twin, a simulation of whether each of the plurality of persons takes a behavior for the policy, so as to identify a route to which each of the plurality of persons is allocated in an option of the conditional branch, and
the generating generates information that indicates a prediction result of the policy that includes an identified route to which each of the plurality of identified persons is allocated.

10. An information output method for causing a computer to execute a process comprising:
identifying a flow of persons in a route that includes a plurality of options by using a behavior selection model that indicates which behavior a person selects for a policy;
generating information that indicates a prediction result of the policy based on an identified flow of persons; and
outputting generated information of a prediction result of policy to a display screen.

11. The information output method according to claim 10, wherein
the computer is caused to further execute a process of acquiring data related to the person,
the identifying includes a process of, when acquired data related to a person is input to a policy model, identifying a route to a terminal node through nodes and edges by using a behavior selection model that indicates which behavior the person selects for the policy, and
the generating includes a process of generating information that indicates a prediction result of policy related to a service to be provided to the person based on an identified route.

12. The information output method according to claim 10, wherein
the identifying includes a process of predicting a number of persons who use a service in each of a plurality of routes to terminal nodes branched with an intermediate node as a starting point, based on a behavior selection model that indicates which behavior a person selects for the policy, and
the generating includes a process of generating information that indicates a prediction result of policy in which a predicted number of persons and a service to be provided to the person are associated with each other.

13. The information output method according to claim 10, wherein
the computer is caused to further execute a process of acquiring a policy model which has a flow structure constituted by nodes and edges, and in which a behavior selection model that indicates which behavior a person selects for the policy is associated with an intermediate node of the flow structure, and a service to be provided to the person is associated with an intermediate node or a terminal node of the flow structure,
the identifying includes a process of identifying a route to a terminal node by tracing nodes and edges of the flow structure based on the behavior selection model when data related to a person to be analyzed is input to an acquired policy model,
the generating includes a process of generating information that indicates a prediction result of policy in which a number of persons flowing in an identified route and a service to be provided to the person are associated with each other, and
the outputting includes a process of outputting generated information that indicates a prediction result of policy to the display screen.

14. The information output method according to claim 10, wherein the identifying identifies a number of persons flowing through a node in a flow graph based on the behavior selection model when acquired data related to a person is input to a policy model,
the generating generates an image in which edges are made thick based on an identified number of persons, and
the outputting displays a generated image on the display screen.

15. An information processing device comprising:
a control unit configured to perform a process of:
identifying a flow of persons in a route that includes a plurality of options by using a behavior selection model that indicates which behavior a person selects for a policy;
generating information that indicates a prediction result of the policy based on an identified flow of persons; and
outputting generated information of a prediction result of policy to a display screen.
